# EUROPÄISCHE PATENTSCHRIFT

(11) **EP 2 217 619 B1**
(45) Veröffentlichungstag und Bekanntmachung des Hinweises auf die Patenterteilung: **07.11.2012**
(21) Anmeldenummer: 08859641.6
(22) Anmeldetag: 12.12.2008
(51) Int. Cl.: C07K 14/525, A61K 38/19

(54) **ZYKLISCHES, CYSTEIN-FREIES PROTEIN**
CYCLIC, CYSTEIN-FREE PROTEIN
PROTÉINE CYCLIQUE EXEMPTE DE CYSTÉINE

(30) Priorität: 12.12.2007 AT 20142007
(43) Veröffentlichungstag der Anmeldung: 18.08.2010
(73) Patentinhaber: Apeptico Forschung Und Entwicklung Gmbh, 1050 Wien (AT)
(72) Erfinder: FISCHER, Bernhard, A-1160 Wien (AT); LUCAS, Rudolf, B-2630 Aartselaar (BE)
(74) Vertreter: Schwarz, Albin
(86) Internationale Anmeldenummer: PCT/AT2008/000448
(87) Internationale Veröffentlichungsnummer: WO 2009/073909

(56) Entgegenhaltungen:
- EP-A- 1 452 868
- WO-A-00/09149
- WO-A-90/06945
- PARASTOO HAZEMI ET AL: "Essential structural features of TNF-alpha lectin-like domain derived peptides for activation of amiloride-sensitive sodium currecnt in A549 cells", JOURNAL OF MEDICINAL CHEMISTRY, AMERICAN CHEMICAL SOCIETY, vol. 53, no. 22, 27 October 2010 (2010-10-27), pages 8021-8029, XP002638326, DOI: 10.1021/JM100767P

### SEQUENCE LISTING

<110> Fischer, Bernhard
<120> zyklisches und Cystein-freies Peptid
<130> B 12159
<140>
   <141> 2007-12-12
<160> 3
<170> PatentIn version 3.3
<210> 1
   <211> 31
   <212> PRT
   <213> Artificial
<220>
   <223> vom Tumor Nekrose Faktor (TNF) abgeleitetes, modifiziertes Peptid
<400> 1
   Val Asn Leu Leu Ser Ala Ile Lys Ser Pro Gly Gln Arg Glu Thr Pro
   1 5 10 15
   Glu Gly Ala Glu Ala Lys Pro Trp Tyr Glu Pro Ile Tyr Leu Gly
   20 25 30
<210> 2
   <211> 19
   <212> PRT
   <213> Artificial
<220>
   <223> vom Tumor Nekrose Faktor (TNF) abgeleitetes, modifiziertes Peptid
<400> 2
   Lys Ser Pro Gly Gln Arg Glu Thr Pro Glu Gly Ala Glu Ala Lys Pro
   1 5 10 15
   Trp Tyr Glu
<210> 3
   <211> 157
   <212> PRT
   <213> Homo sapiens
<400> 3

## Beschreibung

Die vorliegende Erfindung betrifft ein zyklisches, Cystein-freies Protein, das als Medikament verwendet werden kann, z.B. zum Aktivieren epithelialer Ionenkanäle, zur Verbesserung der Lungenfunktion, sowie zur Behandlung von Ödemen, wie Lungenödemen.

Der Flüssigkeitstransport durch Zellschichten und Gewebe beruht primär auf einem osmotischen Gradienten durch einen aktiven vektoriellen Ionentransport, z.B. Natriumtransport. Dieser wird hauptsächlich durch streng regulierten und vital bedeutenden Ionenkanäle, wie z.B. dem Epithelial Natrium- Kanal Komplex (ENaC), bewerkstelligt. Wasser folgt diesem Gradienten passiv, unter anderem durch spezielle Wasserkanäle, wie dem Wasserkanal Aquaporin V. Für das Lungengewebe ist bekannt, dass basolateral an den pumpenden Zellen Na+/K+ ATPasen den vektoriellen Transport von Natrium ins Interstitium betreiben und schließlich die Ionen in die Lymph- und Blutgefässe an. Dieser Transport ist also aktiv und erfolgt unabhängig vom transpulmonalen Druck und der alveolären Proteinkonzentration.

Ein Ödem ist eine pathologische Flüssigkeitsansammlung in einem Organ, wie z.B. der Lunge, aber auch dem Gehirn oder der Haut. Bei einem Ödem in der Lunge spricht man von einem Lungenödem. Das Lungenödem basiert meist auf dem Ungleichgewicht zwischen Flüssigkeitsextravasation und Flüssigkeitsrückresorption. Sehr oft ist auch die Permeabilität des Lungengewebes geschädigt, sodass eine vermehrte Flüssigkeitszufuhr stattfindet, und sich die Flüssigkeit in den Lungenblässchen (Alveolen) ansammelt..

Eine solche Permeabilitätsstörung als Folge eines fehlenden Rücktransportes von Flüssigkeit aus den Lungenbläschen in das Interstitium ist besonders bedeutsam bei der Akuten Lungen Verletzung (engl. Acute Lung Injury, ALI) oder beim Akuten Respiratorischem Distress Syndrom (engl. Acute Respiatory Distress Syndrome, ARDS) oder beim schweren akuten Atemnotsyndrom (SARS), bei der Pneumonie und beim Multiorganversagen. Die Permeabilitätsstörung spielt aber auch eine Rolle bei anderen Lungenerkrankungen, wie Beatmung induzierter Lungenschäden, Lungentransplantationen, Transfusion assoziierter Lungenschäden, therapeutischer Gabe von IL-2 oder Asthma.

Als Ergebnis einer erhöhten Flüssigkeitsansammlung im Gewebe oder Organ, z.B. der Lunge, wird der notwendige Gasaustausch behindert oder völlig eingeschränkt. Es kommt kein Sauerstoff aus der Atemluft in das Blut, sodass durch Sauerstoffmangel lebensgefährliche Organschäden auftreten können.

Es gibt keine generelle Standardtherapie zur Behandlung für das Permeabilitätsödem. Allgemein bemüht man sich Patienten mit einem Lungenödem künstlich zu beatmen, um die Zufuhr von Sauerstoff ins Blut und somit zu den Organen zu gewährleisten.

Aus der DE 38 41 759 sind einzelne, vom Tumor Nekrosis Faktor (TNF) abgeleitete Peptide bekannt.

EP 1 452 868 betrifft ein Verfahren zur Auswahl einer Verbindung, die als Medikament wirksam ist, aus einer Bibliothek von Verbindungen; wobei verschiedene Peptide beschrieben sind.

In WO 90/06945 sind unter anderem Peptide beschrieben, die sich zur Bekämpfung von Krankheiten eignen sollen.

Von Carswell et al. in Proc. Natl. Acad. Sci. USA 72, 3666, 1975 wurde berichtet, dass das Serum von Endotoxin-behandelten Tieren, die zuvor mit dem Mycobacterium-Stamm Calmette-Guerin (BCG) infiziert worden waren, eine hämorrhagische Nekrose bei verschiedenen Tumoren in der Maus bewirkte. Diese Aktivität wurde dem Tumor Nekrose Faktor zugeschrieben. TNF zeigt auch eine zytostatische oder zytotoxische Wirkung gegenüber einer Vielzahl von transformierten Zelllinien in vitro, während normale menschliche und tierische Zelllinien davon nicht betroffen werden (M. R. Ruff et al, Lymphokines, Vol. II, Academic Press Inc., New York, 1981, pp 235-275). Die biochemische Charakterisierung und das Gen für menschlichen TNF wurde bereits beschrieben (D Pennica et al, Nature 312, 724, 1984; Aggarwal, B. B. et al, J. Biol. Chem. 260, 2334-2345, 1985; Nedwin, G.E. et al, Nucl. Acids Res. 13, 6361, 1985).

Aus diesen Daten konnte die folgende Proteinstruktur für den humanen reifen Tumor Nekrose Faktor (TNF) abgeleitet werden:

Weiterhin wurde das TNF-Gen von Rind, Kaninchen und Maus beschrieben (Goeddel D. V. et al., Cold Spring Harbor Symp. Quant. Biol. 51, 597, 1986).

Neben seinen zytotoxischen Eigenschaften ist TNF mit hauptbeteiligt an entzündlichen Reaktionen (J.W. Larrick et al, Pharmac. Res. Vol. 5, No. 3, 129-139, 1988). Im Tiermodell konnte die Beteiligung von TNF beim septischen Schock (Torti F. M. et al, Science 229, 867-869, 1985) und der Graft versus Host Disease (Piguet, P F et al, J. Exp. Med. 166, 1280, 1987) gezeigt werden.

In Lucas R et al, Science (1994) Vol. 263. no. 5148, pp. 814 - 817 ist ein Peptid beschrieben, das von der Region Ser(99) bis Glu(116) des TNF abgeleitet wurde, und welches zur Behandlung von Ödemen vorgeschlagen wird. Dieses Peptid ist auch Gegenstand von WO 00/09149. Um dieses Peptid der WO 00/09149 jedoch nutzbar zu machen, musste artifiziell die Position Pro(100) durch die Aminosäure Cystein und die Position Cys(101) durch die Aminosäure Glycin ersetzt werden. Da das lineare Peptid Ser(99) bis Glu(116) keine erfindungsgemäße Wirkung hatte (Hribar M. et al., Eur. J. Immunol. (1999), Vol.29, 3105-3111; Braun C., J. Immunol. (2005), 175: 3402-3408; Fukuda N. et al. Am J Physiol Lung Cell Mol Physiol (2001) 280: L1258-L1265), musste zusätzlich die Position Glu(116) durch die Aminosäure Cystein ersetzt werden.

Ein solches, in WO00/09149 beschriebenes, Peptid ist zur Herstellung von Medikamenten nicht geeignet, da es zwei Cysteine an den Positionen (100) und (116) enthält, welche bekanntlich in Lösung reduziert werden, wobei die Schwefelbrücke zwischen den Cysteinen zerstört wird und die zyklische Struktur des Peptides sich auflöst, wodurch das Peptid unwirksam gemacht wird.

Es wurde nun überraschend ein zyklisches, Cystein-freies Peptid gefunden, das aus dem reifen Tumor Nekrose Faktors (TNF) abgeleitet ist und interessante biologische Eigenschaften aufweist.

Der reife Tumor Nekrose Faktor (TNF) wie hierin verwendet, ist bevorzugt der humane reife Tumor Nekrose Faktor.

In einem Aspekt stellt die vorliegende Erfindung ein Protein zur Verfügung, das ausgewählt ist aus der Aminosäuresequenz der Region Valin Val(91) bis Glycin Gly(121), oder einem Teil davon, des reifen, humanen Tumor Nekrosis Faktor, mit der Maßgabe, dass das Protein zumindest die Aminosäuresequenz der Region Lysin Lys(98) bis Glutaminsäure Glu(116) umfasst, wobei das Cystein Cys(101) durch ein Glycin ersetzt ist und wobei eine Amidbindung zwischen der Aminogruppe der Seitenkette des Lysins Lys(98) und der Carboxylgruppe der Seitenkette der Glutaminsäure Glu(116) ausgebildet ist ausgebildet ist, und wobei die Proteinstruktur für den humanen reifen Tumor Nekrose Faktor (TNF) die folgende Aminosäuresequenz SEQ ID NO:3 aufweist:

Ein Protein, das gemäß vorliegender Erfindung zur Verfügung gestellt wird, wird hierin auch als "Protein gemäß (nach) vorliegender Erfindung" bezeichnet.

Gemäß vorliegender Erfindung wurden besonders geeignete Proteine mit den Aminosäuresequenzen
**SEQ ID:NO:1** worin eine Amidbindung zwischen der Aminogruppe der Seitenkette des Lysins Lys(8) und der Carboxylgruppe der Seitenkette der Glutaminsäure Glu(26) ausgebildet ist, und
**SEQ ID:NO:2** worin eine Amidbindung zwischen der Aminogruppe der Seitenkette des Lysins Lys(1) und der Carboxylgruppe der Seitenkette der Glutaminsäure Glu(19)

Ein Protein gemäß vorliegender Erfindung kann in geeigneter Weise hergestellt werden, z.B. analog zu einem bekannten Verfahren, oder wie hierin beschrieben, beispielsweise durch chemische Synthese mittels Peptidchemie oder mittels mikrobieller Verfahren. Die Einführung der Amidbindung zwischen der freien Aminogruppe und der freien Carboxylgruppe kann ebenfalls in geeigneter Weise erfolgen, z.B. analog zu einem bekannten Verfahren, oder wie hierin beschrieben.

Ein Protein gemäß vorliegender Erfindung kann in freier Form vorliegen, oder in der Form eines Salzes, z.B. in der Form eines Säureadditionssalzes, wie ein Acetatsalz, oder ein Trifluoressigsäuresalz und in einem weiteren Aspekt stellt die vorliegende Erfindung ein Protein gemäß vorliegender Erfindung in der Form eines Salzes zur Verfügung.

Es hat sich gezeigt, dass ein Protein gemäß vorliegender Erfindung interessante biologische Aktivität zeigt und daher als Medikament verwendet werden kann.

In einem weiteren Aspekt stellt die vorliegende Erfindung ein Protein gemäß vorliegender Erfindung zur Verwendung als ein Medikament zur Verfügung, z.B.
Die Verwendung eines Proteins gemäß vorliegender Erfindung als ein Medikament.

Beispielsweise zeigen biologische Untersuchungen an humanen Zellen, dass ein Protein gemäß vorliegender Erfindung auch im Unterschied zum humanen TNF praktisch keine entzündlichen oder toxischen Eigenschaften aufweisen. Zur Untersuchung werden in laborüblicher Weise humane Immunzellen aus dem Blut mit Protein gemäß vorliegender Erfindung in geringer Konzentration gemischt und inkubiert. Anschlioeßend werden zellfreien Kulturmedium werden mittels üblichen Methoden Markerproteine für Entzündungen bestimmt. Trotz Zugabe eines Proteins gemäß vorliegender Erfindung, z.B. ein Protein der Aminosäuresequenz SEQ ID NO:1 oder SEQ ID NO:2 können solche Entzündungsproteine, wie z.B. der Entzündungsmarker Interleukin-6 (IL-6), nicht nachgewiesen werden.

Ein Protein gemäß vorliegender Erfindung kann in einem Verfahren zur Vermeidung von Entzündungen, z.B. zu Vermeidung der Bildung von Entzündungsmarkem, wie IL-6, bei der medizinischen Anwendung von Proteinen, die vom Tumor Nekrosis Faktor, z.B. vom humanen Tumor Nekrosis Faktor, abgeleitet sind verwendet wird.

Es ist weiterhin eine laborübliche Methode und zum Beispiel in Clunes M.T. et al, J Physiol Volume 557, Number 3, 809-819 (June 15, 2004) beschrieben, die Aktivierung von Ionenkanälen mittels Patch-Clamp Experimenten nachzuweisen. Für Patch-Clamp Untersuchungen von Ionenkanälen wird eine Glaskanüle dünn ausgezogene und mit neutraler Pufferlösung Lösung gefüllt. Die Glaskanüle (Patch-Clamp-Pipette) wird vorsichtig auf eine intakte epitheliale Zelle gedrückt. Unterhalb der Pipette befindet sich ein Stück Membran. Zwischen dem Inneren der Pipette und der Außenlösung entsteht dadurch ein elektrischer Widerstand. In die Pipettenlösung taucht eine Elektrode, die an einen empfindlichen Verstärker angeschlossen ist.

Es ist überraschend zu finden, dass ein Protein gemäß vorliegender Erfindung, z.B. ein Protein mit der Aminosäuresequenz SEQ ID NO:1 oder SEQ ID NO:2, das wie hierin beschrieben durch eine Amidbindung zyklisiert ist, die epithelialen Ionenkanäle aktiviert, was durch die Veränderung der Amplitude des elektrischen Signals nachweisbar ist. Dadurch dass ein Protein gemäß vorliegender Erfindung kein Cystein oder eine Schwefelbrücke enthält, kann ein solches Protein auch nicht reduziert werden.

Zur Simulation eines Akuten Lungenschadens und zur Bildung eines Lungenödems kann in laborüblicher Weise die Lunge von Versuchstieren, z.B. Mäusen oder Ratten, mehrmals mit angesäuerter Kochsalzlösung gespült werden (zum Beispiel gemäß Isik F. et al., Eur J Cardiothorac Surg (2005); 28: 301-305). Dadurch kommt es zur Herabsetzung der Lungenfunktion. Wird nun in die Lunge der Versuchstiere ein Protein gemäß vorliegender Erfindung, z.B. ein Protein der Aminosäuresequenz SEQ ID NO:1 oder SEQ ID NO:2, das wie hierin beschrieben durch eine Amidbindung zyklisiert ist, als Nebel oder in wässriger Lösung injiziert, so kommt es innerhalb von 3 bis 5 Stunden zu einer deutlichen Verbesserung der Lungenfunktion, angezeigt durch gestiegenen Sauerstoffgehalt im arteriellen Blut.
Somit kann ein Protein gemäß vorliegender Erfindung zur Behandlung von Ödemen, wie Lungenödemen, verwendet werden.

Die Behandlung von Krankheiten die mit der Lungenfunktion assoziiert sind, schließt z.B. die Aktivierung epithelialer Ionenkanäle, die Verbesserung der Lungenfunktion und/oder die Behandlung von Ödemen, wie Lungenödemen,
die Behandlung
- der Akuter Lungen Verletzung (engl. Acute Lung Injury, ALI),
- des Akuter Respiratorischen Distress Syndrom (engl. Acute Respiatory Distress Syndrome, ARDS),
- des schweren akuten Atemnotsyndrom (SARS),
- der Pneumonie,
- bei Multiorganversagen,
- bei Beatmung induzierter Lungenschäden, Lungentransplantationen, Transfusion assoziierter Lungenschäden, therapeutischer Gabe von IL-2 oder Asthma
ein,
z.B. die Aktivierung epithelialer Ionenkanäle, die Verbesserung der Lungenfunktion und/oder die Behandlung von Ödemen, wie Lungenödemen.

In einem anderen Aspekt stellt die vorliegende Erfindung ein Protein gemäß vorliegender Erfindung zur Verwendung in der Behandlung von Lungenödemen zur Verfügung.

Ein Patient, wie hierin verwendet, schließt Säugetiere, z.B. Menschen ein.

Ein Protein gemäß vorliegender Erfindung kann in der Form einer pharmazeutischen Zubereitung verabreicht werden.

In einem anderen Aspekt stellt die vorliegende Erfindung eine pharmazeutische Zubereitung zur Verfügung, die dadurch gekennzeichnet ist, dass sie ein Protein gemäß vorliegender Erfindung umfasst, z.B. in Verbindung mit zumindest einem pharmazeutisch akzeptablen Hilfsstoff, wie Träger oder Verdünnungsmittel, beispielsweise Füllstoffe, Bindemittel, Verbesserer der Fließeigenschaften, Gleitmittel, Geschmacksstoffe, Zucker oder Süßstoffe, Geruchsstoffe, Konservierungsstoffe, stabilisierend wirkende Stoffe, Netzmittel, Emulgatoren, Lösungsvermittler, Salze zur Regulierung des osmotischen Drucks und/oder Puffer(mischungen);

Die geeignete Menge eines Proteins gemäß vorliegender Erfindung zur Behandlung von Krankheiten wird natürlich sehr von verschiedenen Parametern abhängen, beispielsweise der chemischen Natur und der Pharmakokinetik des verwendeten Proteins, dem individiuellen Patienten, der zu behandelnden Krankheit, der Art der Anwendung, aber eine erfolgreiche tägliche Dosis in größeren Säugetieren schließt beispielsweise eine Menge von 0.0001 g bis 1.5 g ein, z.B. 0.001 mg/kg Körpergewicht bis etwa 20 mg/kg Körpergewicht ein.
Die Anwendung kann pulmonal oder parenteral erfolgen und erfolgt bevorzugt parenteral. Eine pharmazeutische Zubereitung gemäß vorliegender Erfindung kann in geeigneter Weise hergestellt werden, z.B. analog einer bekannten Methode, z.B. durch Misch-, Granulier-, Beschichtungs-, Lösungs-, Lyophilisierungsverfahren.

### Beschreibung der Abbildungen

Fig. 1A zeigt das HPLC Chromatogramm des Proteins mit der Aminosäuresequenz SEQ ID NO:1. Einheiten: y-Achse: Absorption in mAU; x-Achse: Zeit in Minuten.
Fig._1B zeigt das HPLC Chromatogramm des Proteins mit der Aminosäuresequenz SEQ ID NO:2. Einheiten: y-Achse: Absorption in mAU; x-Achse: Zeit in Minuten.
Fig. 2A zeigt die Aktivierung von Natrium Ionenkanälen durch ein Protein der Aminosäuresequenz SEQ ID NO:1. Einheiten: y-Achse: Anzahl; x-Achse: Amplitude in pA.
Fig. 2B. zeigt die Aktivierung von Natrium Ionenkanälen durch ein Protein der Aminosäuresequenz SEQ ID NO:2. Einheiten: y-Achse: Anzahl; x-Achse: Amplitude in pA.
Fig. 3A zeigt die Erhöhung des Sauerstoffgehalts im arteriellen Blut nach Verabreichung eines Proteins mit der Aminosäuresequenz SEQ ID NO:1. Einheiten: y-Achse: Sauerstoffgehalt in %; x-Achse: Messzeitpunkt in Minuten.
Fig. 3B zeigt die Erhöhung des Sauerstoffgehalts im arteriellen Blut nach Verabreichung eines Proteins mit der Aminosäuresequenz SEQ ID NO:2. Sauerstoffgehalt in %; x-Achse: Messzeitpunkt in Minuten.

### Beispiel 1: Synthese eines Proteins mit der mit der Aminosäuresequenz SEQ ID NO:1 worin eine Amidbindung zwischen der Aminogruppe der Seitenkette des Lysins Lys(8) und der Carboxylgruppe der Seitenkette der Glutaminsäure Glu(26) ausgebildet ist

Ein Protein mit der Aminosäuresequenz SEQ ID NO:1 wurde mittels Fmoc-Festphasensynthese vollautomatisch in folgenden Schritten synthetisiert:

| **Schritt** | **Prozess** | **Produkt** |
|---|---|---|
| 1 | Kopplung der Aminosäuren | Peptid (Protein) gebunden an die Festphase |
| 2 | Abspaltung von der Festphase | Peptid (Protein) in Lösung |
| 3 | Reinigung | Gereinigtes Peptid (Protein) als TFA-Salz |
| 4 | Reinigung / Salz Austausch . | Gereinigtes Peptid (Protein) als Acetatsalz |
| 5 | Analytische Untersuchung | Gereinigtes Peptid (Protein) |

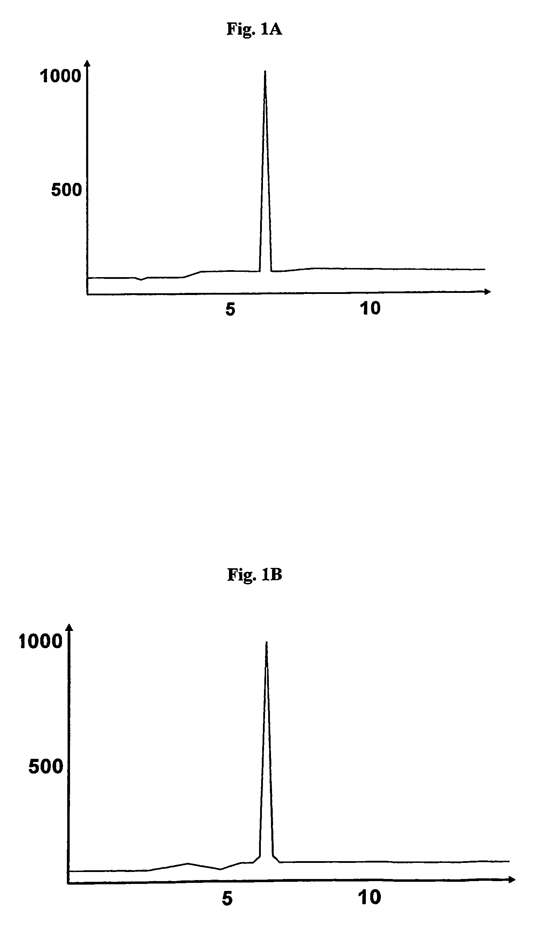

Die Zyklisierung erfolgte durch die Verbindung der epsilon-Aminogruppe des Lysin (Position 8) mit der gamma-Karboxylgruppe der Glutaminsäure (Position 26) unter Bildung einer Amidbindung. Dies erfolgt zum Beispiel dadurch, dass die gamma-Karboxylgruppe der Glutamingruppe mittels Dicyclohexylcarbodiimid (DCC) in einen Aktivester überführt wird, welcher anschließend spontan mit der epsilon-Aminogruppe des Lysin unter Ausbildung eines Ringschusses im Protein reagiert.
Anschließend wurde das Protein mittels Reverser HPLC untersucht, wobei das Ergebnis, wie in Fig. 1A gezeigt, erhalten wurde:

### Beispiel 2: Synthese eines Proteins mit der Aminosäuresequenz SEQ ID NO:2 worin eine Amidbindung zwischen der Aminogruppe der Seitenkette des Lysins Lys(1) und der Carboxylgruppe der Seitenkette der Glutaminsäure Glu(19) ausgebildet ist

Ein Protein mit der Aminosäuresequenz SEQ ID NO:2 wurde mittels Fmoc-Festphasensynthese vollautomatisch in folgenden Schritten synthetisiert

| Schritt | Prozess | Produkt |
|---|---|---|
| 1 | Kopplung der Aminosäuren | Peptid (Protein) gebunden an die Festphase |
| 2 | Abspaltung von der | Peptid (Protein) in Lösung |
| | Festphase | |
| 3 | Reinigung | Gereinigtes Peptid (Protein) als TFA-Salz |
| 4 | Reinigung / Salz Austausch / oxidative Zyklisierung | Gereinigtes Peptid (Protein) als Acetatsalz |
| 5 | Analytische Untersuchung | Gereinigtes Peptid (Protein) |

Die Zyklisierung erfolgte durch die Verbindung der epsilon-Aminogruppe des Lysin (Position 1) mit der gamma-Karboxylgruppe der Glutaminsäure (Position 19) unter Bildung einer Amidbindung. Dies erfolgt zum Beispiel dadurch, dass die gamma-Karboxylgruppe der Glutamingruppe mittels Dicyclohexylcarbodiimid (DCC) in einen Aktivester überführt wird, welcher anschließend spontan mit der epsilon-Aminogruppe des Lysin unter Ausbildung eines Ringschusses im Protein reagiert.
Anschließend wurde das Protein mittels Reverser HPLC untersucht, wobei das Ergebnis wie in Fig. 1 B gezeigt erhalten wurde:

### Beispiel 3: Zellkultur

Die elektrophysiologischen Experimente wurden an humanen H441Zellen durchgeführt. H441 Zellen sind humane Lungenepithelzellen, die an der Diffusion von Wasser und Elektrolyten in der Lunge beteiligt sind.
Die H441 Zellen wurden von der American Tissue Culture Collection bezogen und in üblichen Zellkulturgefäßen in RPMI 1640 Medium (Invitrogen) kultiviert. Das Zellkulturmedium enthielt zusätzlich 4,5 g/Liter Glukose, 1% Penicillin/Streptomycin und 5% fötales Kälberserum.
Für die Patch Clamp Experimente wurden die Zellen auf Glasplättchen übertragen.

### Beispiel 4: Aktivierung von Ionenkanälen humaner Epithelzellen durch Proteine mit den Aminosäuresequenzen SEQ ID NO:1 und SEQ ID NO:2

Makroskopische Ströme und Einzelkanalströme wurden von H441 Zellen in der "whole cell" und "cell-attached" Konfiguration der"Patch-clamp" Technik (Hamill et al, Pflugers Arch. 1981, 391(2):85-100, (1981) abgeleitet.

Zur Messung einzelner Ionenkanäle wurden die Proteine in einer Lösung aus 135 mM Na-Gluconate, 15 mM NaCl, 5 mM KCl, 1 mM MgCl₂, 2 mM CaCl₂, 5 mM Glucose, 10 mM Hepes, pH 7.4 gelöst und in die Patch Pipette gefüllt. Das Zellmembranpotenzial wurde depolarisiert zu 0 mV mittels der Depolarisationslösung aus 140 m KCl, 15 mM NaCl, 5mM MgCl₂, 10 mM Hepes, pH 7.4. Während der Messungen wurde eine Spannung (Patch) von -100 V eingestellt

Dieses Protokoll wurde unter Zugabe von Proteinen mit den Aminosäuresequenzen SEQ ID NO:1 oder SEQ ID NO:2, sowie dem Natriumkanal-Inhibitor Amilorid durchgeführt. Die so erhaltenen Stromableitungen wurden gespeichert und mit Hilfe des Programms PCLAMP 6.0 analysiert.

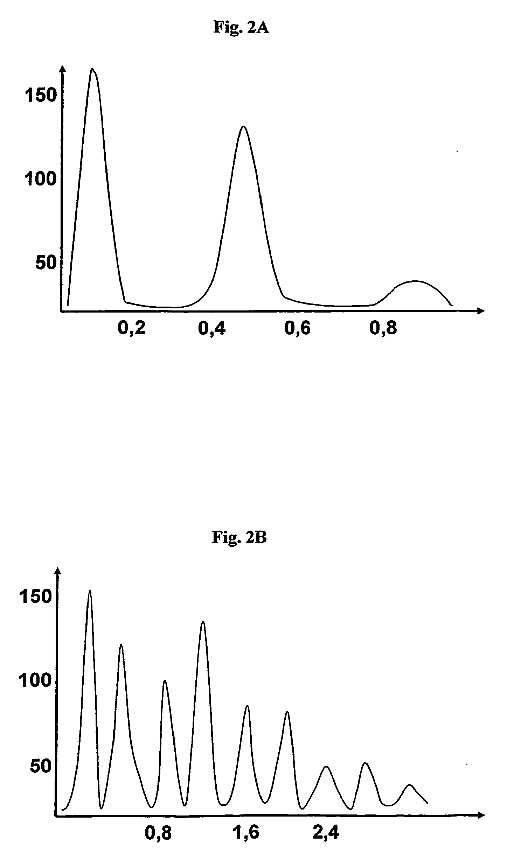

Die Resultate, in denen die Aktivierung der Natrium lonenkanäle durch die Proteine mit den Aminosäuresequenzen SEQ ID NO:1 und SEQ ID NO:2 gezeigt wird, sind aus Fig. 2A und Fig. 2B ersichtlich.

### Beispiel 5: Experimentelle Tierstudie Lungenödem

Männliche Wistar Ratten (Gewicht 250 g bis 350 g) werden mit Rompun® (0,02 ml/100g) und Ketavet® (0,1 ml/100g) anästhesiert. Die Beatmung erfolgt mit einem Zyklus von 72 Stößen / Minute, bei einer Einatmungszeit von 0,2 Sekunden und einer Ausatmungszeit von 0,5 Sekunden. Die Körpertemperatur beträgt durchschnittlich 37°C bis 39°C. Im Normalzustand beträgt der PaO2 (arterieller Sauerstoffpartialdruck) 500 bis 550 mm Hg. Zur Simulation eines Akuten Lungenschadens und zur Bildung eines Lungenödems wird die Lunge 7 bis 9 mal mit angesäuerter Kochsalzlösung (pH 5) gespült.

Nach einer Stunde werden die Proteine mit der Aminosäuresequenz SEQ ID NO:1 oder SEQ ID NO:2, gelöst in steriler Kochsalzlösung, intratracheal als Nebel verabreicht (Maximales verabreichtes Volumen 0,5 ml).

In einem Abstand von jeweils 60 Minuten wird den Tieren arterielles Blut entnommen (0,1 ml) und der Sauerstoffgehalt in % zum Normalwert bestimmt.

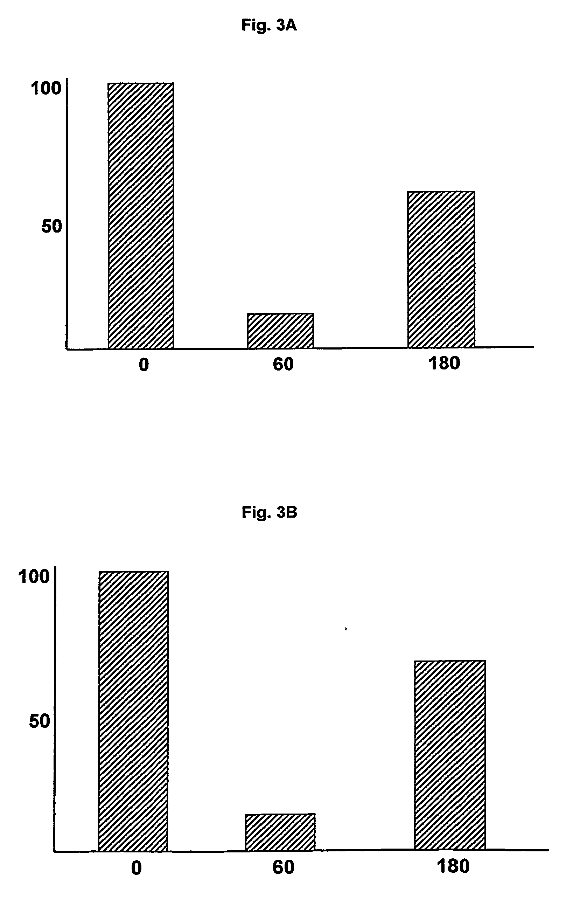

Nach Verabreichung eines Proteins mit der Aminosäuresequenz SEQ ID NO:1 oder SEQ ID NO:2 ist der Sauerstoffanteil im Blut erhöht, wie aus Fig. 3A oder Fig.3B ersichtlich, siehe auch Beispiel 6.

### Beispiel 6: Verbesserung der Lungenfunktion durch Proteine mit den Aminosäuresequenzen SEQ ID NO:1 und SEQ ID NO:2

Der Nachweis der stimulierenden Wirkung eines Proteins gemäß vorliegender Erfindung, z.B. eines Proteins mit der Aminosäuresequenz SEQ ID NO:1, SEQ ID NO:2 oder SEQ ID NO:3, auf die Lungenfunktion erfolgt mittels tierexperimentellen Studien, in welchem ein Lungenödem induziert wird. Die experimentelle Vorgehensweise ist im Beispiel 5 beschrieben.

Zur intratrachealen Inhalation werden jeweils 125 µg Protein in 150 mM Kochsalzlösung pH 7,3 aufgelöst. Der Sauerstoffgehalt des arteriellen Blutes wird unmittelbar vor der Spülung der Lungen, 60 Minuten nach der Spülung der Lungen und 180 Minuten nach Spülen der Lunge gemessen.

Der Sauerstoffgehalt unmittelbar vor dem Lungenspülen wird mit 100% festgelegt. 60 Minuten nach dem jeweils letzen Lungenspülen beträgt der Sauerstoffgehalt im Blut durchschnittlich nur 20%. Innerhalb von 3 Stunden erhöhte sich der prozentuale Sauerstoffgehalt auf Werte von
62% bei Behandlung mit einem Protein mit der Aminosäuresequenz SEQ ID NO:1, bzw.
75% bei Behandlung mit einem Protein mit der Aminosäuresequenz SEQ ID NO:2.

Ohne Zugabe von Protein kommt es innerhalb von 180 Minuten nach Lungenspülung zu keiner Verbesserung der Lungenfunktion (Sauerstoffgehalt 20%).

Die Resultate sind dargestellt in
- Fig. 3A für ein Protein mit der Aminosäuresequenz SEQ ID NO:1,
- Fig. 3B für ein Protein mit der Aminosäuresequenz SEQ ID NO:2.

### Beispiel 7: Bestimmung von Entzündungsparametern

Frisches menschliches Blut reagiert sehr empfindlich auf pro-entzündliche Moleküle, u.a. mit der Freisetzung des Entzündungsmarkers Interleukin-6 (IL-6). Zur Bestimmung der proentzündlichen Reaktion, wurden menschliche Frischblutproben in Konzentrationen des Proteins mit der Aminosäuresequenz SEQ ID NO:2 in folgenden Konzentrationen von 1 ng/ml bis 10 µg/ml inkubiert. Nach einer Inkubation von 24 Stunden bei 37°C wurde in der Lösung der Entzündungsmarker Interleukin-6 mittels ELISA quantitativ bestimmt. Als Positivkontrolle diente LPS (Konzentrationen 3 ng/ml und 100 ng/ml).

Dabei wurden die Messdaten, die in TABELLE 1 angegeben sind und die den Einfluss des Peptides Protein mit der Aminosäuresequenz SEQ ID NO:2 im Vergleich mit LPS auf die Freisetzung des Entzündungsmarkers Interleukin-6 aus Blutzellen erhalten:

**Tabelle 1**

| **Konzentration des Proteins bzw. LPS** | **Protein SEQ ID NO:2** | **Positivkontrolle "LPS"** |
|---|---|---|
| | **Konzentration Interleukin-6 (pg/ml) (Durchschnitt aus fünf Messungen)** | |
| ohne Proteinzugabe (normaler Blutwert) | weniger als 0,5 | weniger als 0,5 |
| 10 µg/ml | weniger als 0,5 | 195.640 |
| 1 µg/ml | weniger als 0,5 | 108.370 |
| 3 ng/ml | weniger als 0,5 | 34.867 |
| 1 ng/ml | weniger als 0,5 | nicht bestimmt |

Die Messdaten in TABELLE 1 zeigen, dass durch eine Inkubation von menschlichen Immunzellen im Frischblut mit einem Protein der Aminosäuresequenz SEQ ID NO:2 praktisch kein Entzündungsmarker IL-6 beigesetzt und somit keine Entzündungsreaktion ausgelöst wird. Demgegenüber bewirkt eine Inkubation mit LPS als Positivkontrolle eine starke Freisetzung des Entzündungsmarkers Interleukin-6.

## Patentansprüche

1. Protein, ausgewählt aus der Aminosäuresequenz der Region Valin Val(91) bis Glycin Gly(121) des reifen, humanen Tumor Nekrosis Faktor, oder einem Teil davon, mit der Maßgabe, dass das Protein zumindest die Aminosäuresequenz der Region Lysin Lys(98) bis Glutaminsäure Glu(116) umfasst, wobei das Cystein Cys(101) durch ein Glycin ersetzt ist und wobei eine Amidbindung zwischen der Aminogruppe der Seitenkette des Lysins Lys(98) und der Carboxylgruppe der Seitenkette der Glutaminsäure Glu(116) ausgebildet ist, und wobei die Proteinstruktur für den humanen reifen Tumor Nekrose Faktor (TNF) die folgende Aminosäuresequenz SEQ ID NO:3 aufweist:

2. Protein nach Anspruch 1, mit der Aminosäuresequenz SEQ ID:NO:1 1 (NH₂)Val-Asn-Leu-Leu-Ser-Ala-Ile-**Lys**-Ser-Pro-Gly-Gln-Arg-Glu-Thr-Pro-Glu-Gly-Ala-Glu-Ala-Lys-Pro-Trp-Tyr-**Glu**-Pro-Ile-Tyr-Leu-Gly(COOH), worin eine Amidbindung zwischen der Aminogruppe der Seitenkette des Lysins Lys(8) und der Carboxylgruppe der Seitenkette der Glutaminsäure Glu(26) ausgebildet ist.

3. Protein nach Anspruch 1, mit denAminosäuresequenz SEQ ID:NO:2 (NH₂)**Lys**-Ser-Pro-Gly-Gln-Arg-Glu-Thr-Pro-Glu-Gly-Ala-Glu-Ala-Lys-Pro-Trp-Tyr-**Glu**(COOH), worin eine Amidbindung zwischen der Aminogruppe der Seitenkette des Lysins Lys(1) und der Carboxylgruppe der Seitenkette der Glutaminsäure Glu(19) ausgebildet ist,

4. Protein gemäß einem der Ansprüche 1 bis 3, zur Verwendung als Medikament.

5. Protein gemäß einem der Ansprüche 1 bis 3, zur Verwendung in der Behandlung von Lungenödemen.

6. Pharmazeutische Zubereitung, umfassend ein Protein gemäß einem der Ansprüche 1 bis 3.

## Claims

1. A protein selected from the amino acid sequence of the region valine Val(91) to glycine Gly(121) of the mature human tumour necrosis factor, or a portion thereof, with the proviso that the protein comprises at least the amino acid sequence of the region lysine Lys(98) to glutamic acid Glu(116), with the cysteine Cys(101) being replaced by a glycine and an amide bond being formed between the amino group of the side chain of the lysine Lys(98) and the carboxyl group of the side chain of the glutamic acid Glu(116), and wherein the protein structure of the mature human tumour necrosis factor (TNF) comprises the following amino acid sequence SEQ ID NO:3:

2. Protein according to claim 1, comprising the amino acid sequence SEQ ID:NO:1 (NH₂)Val-Asn-Leu-Leu-Ser-Ala-Ile-**Lys**-Ser-Pro-Gly-Gln-Arg-Glu-Thr-Pro-Glu-Gly-Ala-Glu-Ala-Lys-Pro-Trp-Tyr-**Glu**-Pro-Ile-Tyr-Leu-Gly(COOH), wherein an amide bond is formed between the amino group of the side chain of the lysine Lys(8) and the carboxyl group of the side chain of the glutamic acid Glu(26).

3. Protein according to claim 1, comprising the amino acid sequence SEQ ID:NO:2 (NH₂)**Lys**-Ser-Pro-Gly-Gln-Arg-Glu-Thr-Pro-Glu-Gly-Ala-Glu-Ala-Lys-Pro-Trp-Tyr-**Glu**(**COOH**), wherein an amide bond is formed between the amino group of the side chain of the lysine Lys(1) and the carboxyl group of the side chain of the glutamic acid Glu(19).

4. Protein according to any of claims 1 to 3 for use as a medicament.

5. Protein according to to any of claims 1 to 3 for use in the treatment of pulmonary oedemas.

6. Pharmaceutical preparation, comprising a protein according to any of claims 1 to 3.

## Revendications

1. Protéine sélectionnée parmi les séquences d'acides aminés de la région qui s'étend entre la valine Val(91) et la glycine Gly(121) du facteur mature humain de la nécrose des tumeurs ou une partie de ce dernier, avec la condition que la protéine comporte au moins la séquence d'acides aminés de la région qui s'étend entre la lysine Lys(98) et l'acide glutamique Glu(116), la cystéine Cys(101) étant remplacée par une glycine et une liaison amide étant formée entre le groupe amino de la chaîne latérale de la lysine Lys(98) et le groupe carboxyle de la chaîne latérale de l'acide glutamique Glu(116),
la structure protéique du facteur humain mature de la nécrose de tumeurs (TNF) présentant la séquence d'acides aminés SEQ ID NO:3 ci-dessous :

2. Protéine selon la revendication 1, présentant la séquence d'acides aminés SEQ ID:NO:1 dans laquelle une liaison amide est formée entre le groupe amino de la chaîne latérale de la lysine Lys(8) et le groupe carboxyle de la chaîne latérale de l'acide glutamique Glu(26).

3. Protéine selon la revendication 1, présentant la séquence d'acides aminés SEQ ID:NO:2 dans laquelle une liaison amide est formée entre le groupe amino de la chaîne latérale de la lysine Lys(1) et la groupe carboxyle de la chaîne latérale de l'acide glutamique Glu(19).

4. Protéine selon l'une des revendications 1 à 3, destinée à être utilisée comme médicament.

5. Protéine selon l'une des revendications 1 à 3, destinée à être utilisée dans le traitement de l'oedème des poumons.

6. Préparation pharmaceutique comprenant une protéine selon l'une des revendications 1 à 3.
